(19) Europäisches Patentamt / European Patent Office / Office européen des brevets

(11) **EP 4 693 185 A1**

(12) **EUROPEAN PATENT APPLICATION**

(43) Date of publication:
**11.02.2026 Bulletin 2026/07**

(21) Application number: **24193264.9**

(22) Date of filing: **07.08.2024**

(51) International Patent Classification (IPC):
**G06T 7/521** (2017.01)   **G06T 7/55** (2017.01)
**A61C 9/00** (2006.01)   **G06F 3/01** (2006.01)
**G06T 17/00** (2006.01)

(52) Cooperative Patent Classification (CPC):
**A61C 9/0053; A61B 1/00188; A61B 1/00194;
G06F 3/017; G06T 7/55; G06T 17/00; G16H 30/20;
G16H 30/40; G16H 40/63; G16H 50/50;**
G06T 2210/41

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **3Shape A/S
1060 Copenhagen K (DK)**

(72) Inventors:
• HOFFGAARD, Simon
  **1060 Copenhagen K (DK)**
• LANGE, Thomas
  **1060 Copenhagen K (DK)**

(74) Representative: **Guardian
IP Consulting I/S
Diplomvej, Building 381
2800 Kgs. Lyngby (DK)**

(54) **METHOD AND SYSTEM FOR PROVIDING DYNAMIC ZOOM ASSISTANCE DURING SCANNING OF A DENTAL OBJECT**

(57)    A computer-implemented method for providing dynamic zoom assistance during scanning process of a dental object (108) is disclosed. The method comprises obtaining light information reflected from the dental object (108) inside an oral cavity by scanning the dental object (108) with an intraoral scanner (101). The method further comprises generating a digital 3D model (103) of the dental object (108) based on the obtained light information, displaying, on a graphical user interface (102), the digital 3D model (103) in a first zoom state. Further, the method comprises detecting that, as a result of motion of the intraoral scanner (101), the generating of the digital 3D model (103) satisfies a criterion, and transitioning from the first zoom state of the digital 3D model (103) to a second zoom state of the digital 3D model (103) based on the detecting step.

200

| Obtaining light information reflected from a dental object by an intraoral scanner | 201 |
| Generating a digital 3D model of the dental object based on the obtained light information | 202 |
| Displaying, on a GUI, the digital 3D model in a first zoom state | 203 |
| Detecting that, as a result of motion of the intraoral scanner, generating of the digital 3D model satisfies a criterion | 204 |
| Transitioning from the first zoom state of the digital 3D model to a second zoom state of the digital 3D model | 205 |

**FIG. 2**

EP 4 693 185 A1

## Description

## Technical field

The disclosure relates to a computer-implemented method and system for providing dynamic zoom assistance during scanning of a dental object. In particular, the disclosure relates to a computer-implemented method and system allowing transitioning between different zoom states of a digital 3D model of the dental object as a result of motion of an intraoral scanner.

## Background

Development of intraoral scanning techniques has been instrumental in the transition to modern digital dentistry. Use of intraoral 3D scanners allows dental practitioners to accurately and quickly capture dental situation of a patient, which may then be visualized on a display as a digital three-dimensional (3D) model. Obtained digital 3D model may thus serve as a digital impression of teeth, offering numerous advantages over a classical physical impression of teeth.

During the scanning process a user may fail to scan completely all regions of the patient's teeth, which may result in generation of an incomplete digital 3D model with missing scan data. This missing scan data may be visually indicated on the digital 3D model, but often it corresponds to very small surfaces of teeth, thus the user may struggle to visually determine the exact part of the digital 3D model that may require additional scanning. In such cases the user may want to command zooming in of the digital 3D model so that the missing scan data can be more easily observed and captured. In general, the user may want to command zooming in and/or zooming out of the digital 3D model during the scanning process, to improve the efficiency of the scanning process and the quality of the generated digital 3D model.

Therefore there is a need to develop methods and systems that will enable, based on the user command, a clear user guidance towards the part with missing information, all while still offering a smooth and continuous scanning process.

The present disclosure addresses prospects to utilize the capabilities of the intraoral 3D scanner systems to dynamically vary zoom levels of the digital 3D model being generated and thereby to clearly indicate the part of the digital 3D model with missing information.

## Summary

In an embodiment, a computer-implemented method for providing dynamic zoom assistance during scanning process of a dental object is provided, the method comprising:

- obtaining light information reflected from the dental object inside an oral cavity by scanning the dental object with an intraoral scanner,
- generating a digital 3D model of the dental object based on the obtained light information,
- displaying, on a graphical user interface, the digital 3D model in a first zoom state,
- detecting that, as a result of motion of the intraoral scanner, generating of the digital 3D model satisfies a criterion,
- transitioning from the first zoom state of the digital 3D model to a second zoom state of the digital 3D model based on the detecting step.

In a further embodiment, a computer-implemented method for providing dynamic zoom assistance during scanning process of the dental object is provided, the method comprising:

- obtaining light information reflected from the dental object inside the oral cavity by scanning the dental object with the intraoral scanner,
- generating the digital 3D model of the dental object based on the obtained light information,
- displaying, on the graphical user interface, the digital 3D model in the first zoom state,
- transitioning from the first zoom state of the digital 3D model to the second zoom state of the digital 3D model based on the motion of the intraoral scanner.

An effect of the methods according to the disclosure is that a zoom state of the generated digital 3D model may be varied based on the motion of the intraoral scanner, thereby revealing a part of the digital 3D model with missing information.

Expression "3D" throughout the present disclosure refers to a term "three-dimensional". Term "digital 3D model of a dental object" refers to a digital, three-dimensional, computer-generated representation of an object inside the patient's mouth, such as a tooth, gingiva or palate. Similarly, expression "2D" throughout the present disclosure refers to a term "two-dimensional".

Such digital 3D model of the dental object may accurately represent the actual dental object being scanned. That means that teeth, teeth surfaces, restorations and/or gingiva on the digital 3D model may correspond to those inside the oral cavity.

The digital 3D model may be constructed by a processor, based on the light information reflected from the dental object inside the oral cavity by scanning the dental object with the intraoral scanner. The intraoral scanner throughout the disclosure is also referred to as the intraoral 3D scanner. The digital 3D model can be stored in a memory of a computer system, for example in a Standard Triangle Language (STL) format, Standard for the Exchange of Product Data (STEP) format, OBJ format or similar format for storing or printing 3D objects.

The digital 3D model can be received or accessed by the processor. The digital 3D model may usually be displayed on the graphical user interface of a display screen in form of a 3D mesh. The 3D mesh may be comprised of individual facets, for example triangular facets while each facet may comprise, for example, three mutually connected vertices. Alternatively, the digital 3D model may be displayed as a point cloud comprising points, a graph comprising nodes and edges, a volumetric representation comprising voxels, or any other suitable 3D representation form.

The method according to the disclosure may comprise obtaining the light information reflected from the dental object inside the oral cavity by scanning the dental object with the intraoral scanner. In this way, a plurality of two-dimensional (2D) images may be captured based on which sub-scans can be generated, representing surface geometry of a part of the dental object being scanned. Sub-scans may be understood as partial digital 3D models that may be fused together as the scanning process progresses such that the complete digital 3D model is generated at the end of the scanning process.

The method may further comprise generating the digital 3D model of the dental object based on the obtained light information. The light information may comprise information about the geometry and surface texture of the dental object being scanned. The obtained light information may be in form of the plurality of 2D images that may be processed into the sub-scans. Sub-scans may be then "stitched" together as the scanning process progresses, thereby continuously expanding the digital 3D model rendered on the graphical user interface.

Generation of the digital 3D model based on the obtained light information may occur in real-time.

The method may further comprise displaying, on the graphical user interface, the digital 3D model in the first zoom state. The first zoom state may be a default zoom state in which the user may see, on the graphical user interface, the entire digital 3D model once it has been generated. In the first zoom state the details such as individual facets of the digital 3D model are usually not visible.

The method may further comprise detecting that, as a result of the motion of the intraoral scanner, the generating of the digital 3D model satisfies the criterion, and transitioning from the first zoom state of the digital 3D model to the second zoom state of the digital 3D model based on the detecting step.

Algorithm defining the transitioning of zoom states of the digital 3D model, such as transitioning from the first zoom state to the second zoom state, may be referred to as a zoom transition algorithm.

As an effect of the intraoral scanner motion, the zoom state of the digital 3D model may be altered. The user may desire change of the zoom state, for example to increase the zoom level in order to identify exact region, such as facets, of the digital 3D model where scan data is missing.

In an embodiment the criterion may comprise measuring a volume of a part of the digital 3D model generated within a predetermined time and comparing the measured volume to a threshold volume. The threshold volume may be defined empirically.

The volume of the part of the digital 3D model generated within the predetermined time may be computed as a volume of a bounding box enclosing the part of the digital 3D model generated within the predetermined time. The bounding box may be understood as a 3D box enclosing the part of the digital 3D model with a smallest volume.

In another example, the volume of the part of the digital 3D model generated within the predetermined time may be determined by calculating signed volumes of tetrahedrons which are formed by joining the vertices of facets of the part of the digital 3D model with an arbitrary point and summing up the calculated volumes.

To determine the desired volume, a sum of signed volumes of tetrahedrons defined by three vertices of each facet may be determined. The origin of the tetrahedrons may be an arbitrary point. To calculate a signed volume of a tetrahedron with vertices $\vec{v_1}, \vec{v_2}, \vec{v_3}$, following formula may be used:

$$\text{Volume} = \frac{1}{6} |\vec{v_1} \cdot (\vec{v_2} \times \vec{v_3})|$$

The predetermined time may be selected as a time interval from 0 to 10 seconds, more preferably from 1 to 4 seconds and most preferably from 2 to 3 seconds. Selecting the predetermined time value may define how reactive the zoom transition algorithm is.

In an example, a further parameter for activating or accelerating the zoom transition algorithm may be introduced. Thus, the criterion may alternatively and/or additionally comprise, measuring the volume of the part of the digital 3D model generated within a second predetermined time and comparing the measured volume to a second threshold volume. The second predetermined time may be lower than the predetermined time and the second threshold volume may be lower than the threshold volume. For example the second predetermined time may be in the range of 0-2 seconds of the scanning process. The second threshold volume may be set to substantially zero cubic millimeters. In this way it may be detected that, within the second predetermined time the intraoral scanner has been stopped and no generation of the digital 3D model is occurring. Hereby an immediate control for the transitioning from the first zoom state of the digital 3D model to the second zoom state of the digital 3D model may be provided to the user. If either of the defined conditions is satisfied, the zoom transition algorithm may be triggered.

A plurality of sub-scans may be registered such that a time and a position of each sub-scan of the plurality of sub-scans is registered. Term "register" may refer to determining the location of incoming sub-scans in the already-generated digital 3D model and expanding the digital 3D model with new 3D information.

The time of registration of each sub-scan may be internally recorded and considered in the criterion for zoom state transition. The position of each sub-scan may refer to a 3D position of a center of mass or a center of volume of the each sub-scan with coordinates in x,y,z coordinate system of the digital 3D model. Assuming uniform mass density of the digital 3D model, the center of mass and the center of volume would be the same point.

Comparing the measured volume to the threshold volume may comprise determining that the measured volume is lower than the threshold volume. This condition may indicate that the user moves the intraoral scanner sufficiently slow such that the zoom state transition may occur.

The criterion may additionally comprise measuring a speed of the center of mass or a center of volume of each sub-scan of the plurality of sub-scans, and comparing the speed to a threshold value for the center of mass speed or a center of volume speed.

The criterion may yet further comprise measuring an acceleration of the center of mass of each sub-scan of the plurality of sub-scans, or measuring an acceleration of the center of volume of each sub-scan of the plurality of sub-scans, and comparing the measured acceleration to a threshold value for the center of mass acceleration or a center of volume acceleration.

The additional criteria of the speed and/or acceleration of the center of mass or the center of volume may improve the sensitivity of the zoom state transition algorithm and provide the user with more control of activating the zoom transition algorithm.

In a further embodiment the criterion may comprise measuring a distance between furthest points of the part of the digital 3D model generated within the predetermined time and comparing the measured distance to a threshold distance. In this way, the measured distance falling below the threshold distance value may represent sufficiently slow movement of the intraoral scanner by the user, and therefore may trigger the zoom state transition.

The furthest points of the part of the digital 3D model may correspond to a point of a first registered sub-scan of the plurality of sub-scans and a point of a last registered sub-scan of the plurality of sub-scans, wherein the plurality of sub-scans are registered within the predetermined time.

The threshold distance may be in a range of 2-10 millimeters, preferably in a range of 4-8 millimeters. Its minimum value may be selected such that it is sufficiently high for the zoom state transition algorithm not to respond too slow. Its maximum value may be selected such that the algorithm does not react too fast, for example immediately on slowing down of the intraoral scanner movement.

In another example, the criterion may comprise measuring distances between furthest points of each two consecutively registered sub-scans of the plurality of sub-scans and determining an average distance of the measured distances, wherein the plurality of sub-scans are registered within the predetermined time. In this case the average distance may be compared to the threshold distance.

In yet a further embodiment, the criterion may comprise determining that a speed of movement of a scan frame is less than a scan frame speed threshold, wherein the scan frame is a 2D frame displayed on the graphical user interface representing a current position of the intraoral scanner, or more specifically its field of view.

The scan frame may be displayed on the graphical user interface together with the digital 3D model, such that the position of the scan frame in relation to the digital 3D model corresponds to the position of the field of view of the intraoral scanner in relation to the dental object being scanned.

The speed of movement of the scan frame may be a speed of a scan frame center point with respect to a center of mass of the digital 3D model within the scan frame.

The method according to an embodiment may further comprise transitioning from a first zoom state of the scan frame to a second zoom state of the scan frame simultaneously with transitioning to the second zoom state of the digital 3D model.

Alternatively, the method may comprise transitioning from the first zoom state of the scan frame to the second zoom state of the scan frame with a time delay compared with transitioning to the second zoom state of the digital 3D model.

In a yet further embodiment, the scan frame may be in a single zoom state during the transitioning to the second zoom state of the digital 3D model.

Transitioning from the first zoom state of the scan frame to the second zoom state of the scan frame may be such that the scan frame remains completely within the graphical user interface. In that way it can be ensured that the scan frame, when in the second zoom state, will not depart from an observable three-dimensional scene.

In an example transitioning from the first zoom state of the scan frame to the second zoom state of the scan frame may comprise determining an intersection of the scan frame and a border of the graphical user interface and adjusting the second zoom state of the scan frame such that the scan frame remains completely within the graphical user interface.

Similarly, in a case where the scan frame is constant, transitioning from the first zoom state of the digital 3D model to the second zoom state of the digital 3D model may comprise determining an intersection of the digital 3D model and the border of the graphical user interface and adjusting the second zoom state of the digital 3D model such that the digital 3D model remains completely within the graphical user interface.

The digital 3D model may be configured to rotate around its center of mass or center of volume during the displaying on the graphical user interface. The digital 3D model may alternatively be configured to rotate around the center of the scan frame during the displaying on the graphical user interface.

Transitioning from the first zoom state of the digital 3D model to the second zoom state of the digital 3D model may enhance visibility of parts of the digital 3D model with missing information.

The parts of the digital 3D model with missing information may be in the form of holes in the digital 3D model. Holes in the digital 3D model may signify lack of scan data captured by the intraoral scanner. The missing information may be represented in other ways on the digital 3D model, for example in form of a color overlay to indicate that the adequate color information of the corresponding part of the digital 3D model is missing for example.

The parts of the digital 3D model with missing information may be colored in a different color compared to the rest of the digital 3D model.

Generally, the missing information, such as the holes, may correspond to the part of the dental object that has not been scanned or that requires additional scanning or re-scanning.

The second zoom state of the scan frame may correspond to the second zoom state of the digital 3D model. In that way a size ratio of scan frame to the digital 3D model may be kept constant.

The second zoom state, or more precisely a level of zoom in the second zoom state, of the digital 3D model may in one example be pre-determined. In another example, the second zoom state may be determined dynamically. For example, the second zoom state may be a function of a size of a hole in the digital 3D model. The second zoom state may be a function of a size of a tooth within the scan frame of the digital 3D model. The second zoom state may also be a function of a size of a display unit comprising the graphical user interface.

A maximum value of the second zoom state of the digital 3D model may be pre-selected or determined such that the scan frame does not become larger than the graphical user interface.

In an example, the second zoom state of the digital 3D model may be greater than the first zoom state of the digital 3D model. By transitioning to the second zoom state of the digital 3D model, the part of the digital 3D model with missing information may be zoomed in. For example, the second zoom state of the digital 3D model may be such that individual facets with missing information may be visible to the user.

In an example, transitioning from the first zoom state of the digital 3D model to the second zoom state of the digital 3D model may zoom in the digital 3D model on a preparation line for a crown, a filling and/or a dental condition lesion.

In an embodiment, the criterion may comprise detecting the preparation line for the crown, the filling and/or the dental condition lesion in the generated digital 3D model.

Transitioning from the first zoom state of the digital 3D model to the second zoom state of the digital 3D model may be based on detecting a reduction in speed of movement of the intraoral scanner in the oral cavity by the user, over a certain time. Generally, the user may be able to command zoom state change based on motioning the intraoral scanner with a speed that is below or above a speed threshold for the predetermined time. Speed of the zoom state change may also vary depending on the motion of the intraoral scanner. For example, the zoom state change with a first speed may occur during a slow-down of the intraoral scanner movement while the zoom state change with a second speed may occur if a complete stop of the intraoral scanner movement is detected.

In one example, the speed of a tip of the intraoral scanner may be measured by a sensor over the predetermined time. The sensor may be an encoder placed inside the intraoral scanner. The measured speed of the intraoral scanner tip may be averaged over the measured predetermined time and, if lower than the speed threshold, the zoom state of the digital 3D model may be changed from the first zoom state to the second zoom state.

The method may further comprise determining that the speed of the tip of the intraoral scanner is higher than the speed threshold and transitioning back to the first zoom state of the digital 3D model.

The method may further comprise determining that the speed of movement of the scan frame is higher than the scan frame speed threshold and transitioning back to the first zoom state of the digital 3D model.

In general, the method according to the disclosure may comprise transitioning, from the second zoom state of the digital 3D model to the first zoom state of the digital 3D model, based on detecting that, as a result of the motion of the intraoral scanner, generating of the digital 3D model satisfies a further criterion. The further criterion may be based on one or more of the same thresholds as the criterion for transitioning from the first zoom state to the second zoom state of the digital 3D model, wherein the motion of the intraoral scanner in this case relates to motioning of the intraoral scanner above the speed threshold for the predetermined time.

According to the disclosure, a computer-implemented method for providing dynamic zoom assistance during scanning process of the dental object is provided, the method comprising:

- obtaining light information reflected from the dental object inside the oral cavity by scanning the dental object with the intraoral scanner,
- generating the digital 3D model of the dental object based on the obtained light information,
- displaying, on the graphical user interface, the digital 3D model in a first zoom state,

- transitioning from the first zoom state of the digital 3D model to a second zoom state of the digital 3D model based on detecting a voice control signal.

In this way, the user may issue a command for zoom state transitioning verbally. This may be beneficial as the movement of the intraoral scanner does not need to be analyzed for triggering the zoom state transition algorithm.

The detected voice control signal may be used solely or in combination with other criteria recited in the disclosure.

According to a further embodiment of the disclosure, a computer-implemented method for providing dynamic zoom assistance during scanning process of the dental object is provided, the method comprising:

- obtaining light information reflected from the dental object inside the oral cavity by scanning the dental object with the intraoral scanner,
- generating the digital 3D model of the dental object based on the obtained light information,
- displaying, on the graphical user interface, the digital 3D model in a first zoom state,
- transitioning from the first zoom state of the digital 3D model to the second zoom state of the digital 3D model based on detecting the user engaging a button on the intraoral scanner.

The button may be a dedicated button which, when engaged, triggers the zoom state transition algorithm. The zoom state transition may be reversed when the button is disengaged.

According to the disclosure a dental scanning system is disclosed, wherein the dental scanning system comprises:

- the intraoral scanner for scanning the dental object inside the oral cavity, the intraoral scanner comprising:
- a projector unit for projecting a pattern of light onto a surface of the dental object,
- an image sensor for acquiring a plurality of images in response to illuminating the surface of the dental object,
- a processor operatively coupled to the intraoral scanner, the processor configured to perform the steps according to any method of the disclosure.

According to the disclosure a dental scanning system is disclosed, wherein the dental scanning system comprises:

- the intraoral scanner for scanning the dental object inside the oral cavity, the intraoral scanner comprising:
- the projector unit for projecting the pattern of light onto the surface of the dental object,

- the image sensor for acquiring a plurality of images in response to illuminating the surface of the dental object,
- the processor operatively coupled to the intraoral scanner, the processor configured for:

- obtaining light information reflected from the dental object inside the oral cavity,
- generating the digital 3D model of the dental object based on the obtained light information,
- displaying, on the graphical user interface, the digital 3D model in the first zoom state,
- detecting that, as the result of the motion of the intraoral scanner, the generating of the digital 3D model satisfies a criterion,
- transitioning from the first zoom state of the digital 3D model to the second zoom state of the digital 3D model based on the detecting step.

The method according to a further embodiment may comprise:

- obtaining light information reflected from the dental object inside the oral cavity by scanning the dental object with the intraoral scanner,
- generating the digital 3D model of the dental object based on the obtained light information,
- displaying, on the graphical user interface, the digital 3D model in the first zoom state,
- detecting a tooth preparation, a filling and/or a crown within the generated digital 3D model,
- transitioning from the first zoom state of the digital 3D model to the second zoom state of the digital 3D model based on the detecting step.

A computer program product is further disclosed, the computer program product comprising instructions which, when executed by a computer, cause the computer to perform the method according to any of the embodiments presented herein.

A computer-readable medium is further disclosed, the computer-readable medium comprising instructions which, when executed by a computer, cause the computer to carry out the method according to any of the embodiments presented herein.

**Brief description of the figures**

FIG. 1 schematically illustrates scanning of an oral cavity with an intraoral scanner and a corresponding digital 3D model displayed on a graphical user interface;

FIG. 2 illustrates steps of the method according to an embodiment;

FIGS. 3A and 3B illustrate a graphical user interface with the digital 3D model in a first zoom state and in a second zoom state, respectively;

FIGS. 4A and 4B illustrate an example of a volume criterion of the generated digital 3D model for transitioning into the second zoom state;

FIGS. 5A and 5B illustrate an example of a distance criterion of the generated digital 3D model for transitioning into the second zoom state;

FIG. 6 illustrates the digital 3D model in the first zoom state and a scan frame;

FIGS. 7A and 7B illustrate transitioning into the second zoom state of the digital 3D model based on detecting a tooth preparation for a crown;

FIG. 8 illustrates an intraoral scanner system.

## Detailed description

In the following description, reference is made to the accompanying figures, which show by way of illustration how the invention may be practiced.

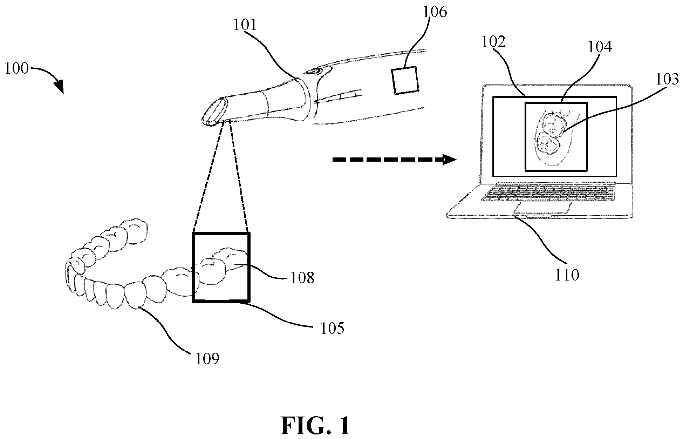

FIG. 1 illustrates a part of an intraoral scanner system 100, namely a handheld intraoral scanner 101 and a graphical user interface 102 which may be a part of a computer device, for example a laptop computer 110. The intraoral scanner 101, also referred to as a scanner or as a 3D intraoral scanner, may be configured for scanning an oral cavity of a patient. As illustrated in figure 1, teeth of lower jaw 109 of the patient's oral cavity may be scanned.

The intraoral scanner 101 may be configured to obtain light information reflected from a dental object 108 inside the oral cavity through a field-of-view 105 of the intraoral scanner 101.

The dental object 108 may be any one tooth or a part thereof, a plurality of teeth, gingiva, a preparation for a crown, a filling, a dental condition lesion, a palate, a restoration or any other object in the oral cavity. The intraoral scanner system 100 may comprise one or more processors 106 configured to process the light information and to generate a digital 3D model 103 of the dental object 108 based on the processed light information. The one or more processors 106 may be located within the intraoral scanner 101 (as illustrated in figure 1), within the computer device displaying the graphical user interface 102 and/or may be a part of a remote computer resource.

A region (surface) of the oral cavity captured by the intraoral scanner 101 may be defined by the field-of-view 105 of the intraoral scanner 101, shown as rectangle in figure 1. The intraoral scanner 101 may receive the light information from the dental object 108 that is within the field-of-view 105 of the intraoral scanner 101. Based on the received light information, the one or more processors 106 may generate, in real time, the digital 3D model 103. The generated digital 3D model 103 may represent the surface of the dental object 108. As a user, for example a dentist, moves the intraoral scanner 101 within the oral cavity, scanner's field-of-view may cover remaining regions of the oral cavity and the digital 3D model 103 may be updated accordingly.

The scanning process may be based on any known scanning principle such as focus scanning, triangulation, confocal scanning, stereo vision, structure from motion.

For example, the intraoral scanner 101 may be based on a focus scanning principle where the intraoral scanner 101 may comprise an optical element, such as a focus lens, configured to move back and forth during scanning to change the focus of the intraoral scanner 101, whereby depth information can be estimated based on a focus measure. A focus scanner is further described in EP 2 442 720 B1, which is hereby incorporated by reference. In some examples, the intraoral scanner 101 may be based on a depth-from-defocus scanning principle, wherein an optical property, such as an aperture, is changed between the acquisition of two images, whereby depth can be estimated by determining the degree of defocus between the two images. A focus scanner may be characterized by a relatively narrow depth-of-field property which may define a volume in which the dental object may be, for it to be in-focus while being scanned. The depth-of-field of the focus scanner may be understood as a depth interval from 0 to a maximum depth-of-field. In an example the interval may be 0 to 20 millimeters, where 20 millimeters is maximum depth-of-field.

In some examples, the intraoral scanner 101 may be based on triangulation principle, wherein at least one camera and a projector unit are positioned such that they form a triangle with respect to a point on the scanned surface. As an example, a projector and a camera may be utilized to determine points in 3D space based on triangulation. Alternatively, the intraoral scanner 101 may comprise two or more cameras viewing the scene or scanned object from two different directions, wherein the cameras are configured to acquire a set of images, wherein a correspondence problem is solved based on triangulation. The correspondence problem generally refers to the problem of ascertaining which parts of one image correspond to which parts of another image. Specifically, the projector unit may be configured to project a plurality of projector rays, which are projected onto a surface of the dental object. In particular, solving the correspondence problem may include the steps of determining image features in the images within a set of images, and further associate said image features with a specific projector ray. Subsequently, the depth of each projector ray may be computed, whereby a 3D representation of the scanned object may be generated. A triangulation scanner may be characterized by a deeper depth-of-field property compared to a focus scanner.

The intraoral scanner 101 may capture a plurality of two-dimensional (2D) images of the dental object 108 which may be converted into sub-scans. A sub-scan generally refers to a digital three-dimensional structure which, when fused with other sub-scans, generated as the scanning progresses, may form the digital 3D model. The process of combining the sub-scans together may be referred to as "stitching" or registration. Registering of the sub-scans may comprise joining together those sub-scans having overlapping portions.

The one or more processors 106 may be configured to display, on the graphical user interface 102, a scan frame 104 that may represent a position of the field-of-view 105 of the intraoral scanner 101. In this way, the user may know exactly what region of the oral cavity is within the scanner's field-of-view 105 by observing the scan frame 104. Thus, movement of the intraoral scanner 101 may result in the corresponding movement of the scan frame 104 on the graphical user interface 102. The scan frame 104 may be configured to move with respect to the digital 3D model 103 in an example. In another example, the scan frame 104 may be stationary while the digital 3D model 103 may be configured to move within the graphical user interface 102.

The scan frame 104 may be preferably displayed overlaying the digital 3D model 103 being rendered, as shown in figure 1. Additionally or alternatively, the scan frame 104 may be displayed separately from the digital 3D model 103 being rendered on the graphical user interface 102 and not overlaying the digital 3D model 103. Such separately displayed scan frame 104 may indicate the position of the field-of-view 105 of the intraoral scanner 101 by showing a 2D image or a plurality of 2D images or a stream of 2D images as they are captured by the intraoral scanner 101. In general, the scan frame 104 may be displayed on the graphical user interface 102 and may indicate the position of the intraoral scanner 101 with respect to the dental object 108 being scanned.

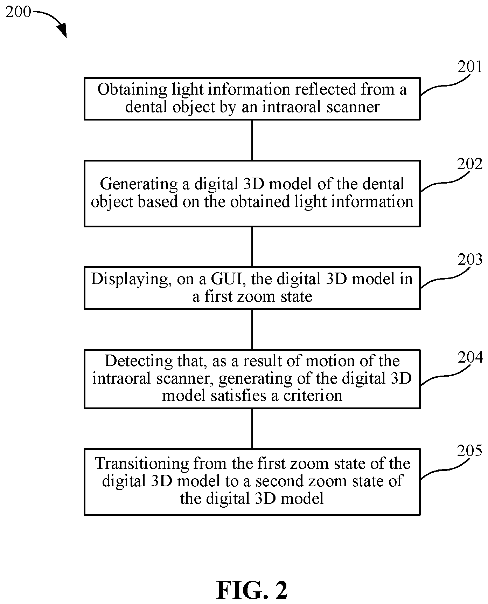

FIG. 2 illustrates a method 200 for providing dynamic zoom assistance during scanning process according to an embodiment.

Step 201 illustrates obtaining light information reflected from the dental object 108 inside the oral cavity by scanning the dental object 108 with the intraoral scanner 101.

The intraoral scanner 101 may be equipped with one or more projector units for projecting a pattern of light onto a surface of the dental object 108. The intraoral scanner 101 may further be equipped with one or more image sensors for acquiring one or more two-dimensional images in response to illuminating the dental object 108.

Step 202 illustrates generating the digital 3D model 103 of the dental object 108 based on the obtained light information.

Generation of the digital 3D model 103 may occur continuously, in real time, during the scanning session. Usually, the digital 3D model 103 may be generated based on sub-scans, wherein the sub-scans are generated based on the acquired two-dimensional images.

The digital 3D model 103 may be updated based on registration of new sub-scans to the digital 3D model 103, wherein the new sub-scans at least partially overlap with the digital 3D model 103. Such continuously generated digital 3D model 103 may be displayed on a graphical user interface 102.

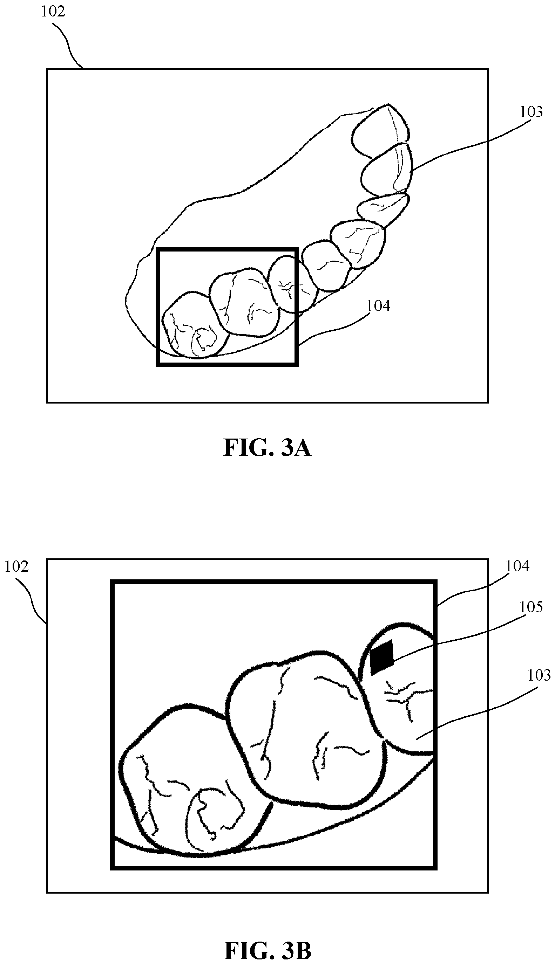

Step 203 of the method 200 illustrates displaying, on the graphical user interface 102, the digital 3D model 103 in a first zoom state (illustrated in FIG. 3A).

Displaying of the digital 3D model 103 may also be referred to as rendering and may occur in real-time as the digital 3D model 103 is being generated.

The first zoom state may refer to a size of the digital 3D model 103 in relation to the graphical user interface 102. The first zoom state may be such that the generated digital 3D model 103 fits in its entirety within the graphical user interface 102. The first zoom state may be such that the user may observe the digital 3D model 103 globally, i.e. that all of the teeth of the digital 3D model 103 fit within the graphical user interface 102 but where finer details of the digital 3D model's surface are not visible. The first zoom state may thus be understood as a default zoom state intended to give the user an overview of the complete 3D model 103.

While performing the scanning process, the user may omit scanning certain parts of the dental object 108 or scan certain parts of the dental object 108 incompletely. This can happen due to a number of reasons, for example due to scanning too fast, due to lack of scanning experience, due to following a sub-optimal scan path etc. As a result, the generated 3D digital model 103 may comprise missing information. The user may be aware of the missing information, for example by observing that the displaying of the digital 3D model 103 does not progress, or does not correspond to the movement of the intraoral scanner 101. However, the user may not be able to determine the exact location of the digital 3D model 103 with the missing information. The user would, in this case, benefit from being able to simply instruct zooming-in of the digital 3D model 103 displayed on the graphical user interface 102, so that the part of the digital 3D model 103 with missing information becomes visible, or to further enhance its visibility. Such command for zooming-in of the digital 3D model 103 may be generated by the user motioning the intraoral scanner 101 that will cause the generating of the digital 3D model 103 to satisfy a criterion, which may be detected by the processor 106 for example.

The step 204 of the method 200 illustrates detecting that, as a result of motion of the intraoral scanner 101, generating of the digital 3D model 103 satisfies the criterion.

Step 205 of the method 200 illustrates transitioning from the first zoom state of the digital 3D model 103 to a second zoom state of the digital 3D model 103 based on the detecting step.

FIG. 3A illustrates the digital 3D model 103 in the first zoom state. The first zoom state may be such that the complete digital 3D model 103, at each moment fully fits within the graphical user interface 102. A scan frame 104, which may be a 2D object, may be displayed on top of the digital 3D model 103 representing a position of a field of view of the intraoral scanner 101. As the user moves the intraoral scanner 101 within the oral cavity, the scan frame 104 may correspondingly move over the digital 3D model 103.

FIG. 3B illustrates the digital 3D model 103 in the second zoom state. The second zoom state, or more specifically its corresponding zoom level, may be greater than the first zoom state. In the second zoom state, the digital 3D model 103 may be zoomed in within the graphical user interface 102 such that an area with missing information is visible, or that its visibility is enhanced compared to the first zoom state.

The missing information may be displayed in several ways. For example, missing information may be represented as a hole 105 in the digital 3D model 103. This hole 105 may not be visible in the first zoom state of the digital 3D model 103. The hole 105 may indicate that scan data is missing for the part of the digital 3D model where the hole 105 is located. By scanning the corresponding area of the oral cavity, the missing scan data may be obtained and the hole 105 may be closed. The missing information may also be represented as a color overlay with a color different to the colors of the rest of the digital 3D model 101. The visible color overlay may indicate that appropriate color information is missing and that the user should re-scan the corresponding part of the oral cavity.

FIG. 3B additionally illustrates the scan frame 104 in a second zoom state compared to the scan frame 104 in a first zoom state illustrated in FIG. 3A. Transitioning from the first zoom state of the scan frame 104 to the second zoom state of the scan frame 104 may occur either simultaneously with transitioning to the second zoom state of the digital 3D model 103 or alternatively with a time delay between. Preferably, this delay would not be visually noticeable.

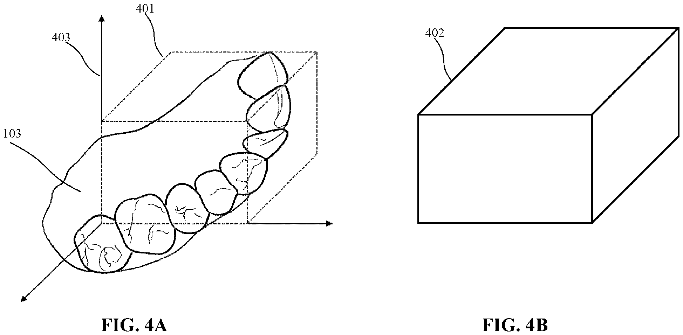

FIGS. 4A and 4B illustrate the generated digital 3D model 103 satisfying a volume criterion for transitioning into the second zoom state.

In this example, the criterion may comprise measuring a volume 401 of a part of the digital 3D model 103 generated within a predetermined time and comparing the measured volume to a threshold volume 402, which may be determined empirically.

The volume 401 of the part of the digital 3D model 103 may be defined by a bounding box enclosing the part of the digital 3D model 103 generated within the predetermined time.

The volume 401 of the part of the digital 3D model 103 may be defined in a coordinate system 403 of the digital 3D model 103.

Registration of the plurality of sub-scans may comprise recording of a time and a position of each sub-scan of the plurality of sub-scans. The time of a sub-scan may refer to a time instance when the sub-scan is generated. The position of the sub-scan may refer to a three-dimensional coordinate of a center of mass or a center of volume of the sub-scan. The center of mass and a center of volume may often be the same point if uniform distribution of mass is considered for the digital 3D model 103.

The criterion may comprise determining that the measured volume 401 is less than the threshold volume 402.

In order to further fine-tune the transitioning from the first zoom state of the digital 3D model 103 to the second zoom state of the digital 3D model 103, additional tuning parameters may be defined.

In one example, the criterion may additionally comprise measuring a speed of the center of mass or a center of volume of each sub-scan of the plurality of sub-scans, and comparing the speed to a threshold speed. This speed of the center of mass or the center of volume may refer to a speed with respect to the scan frame 102.

Additionally or alternatively, the criterion may comprise measuring an acceleration of the center of mass or the center of volume of each sub-scan of the plurality of sub-scans, and comparing the acceleration to a threshold acceleration. This acceleration of the center of mass or the center of volume may refer to an acceleration with respect to the scan frame 102.

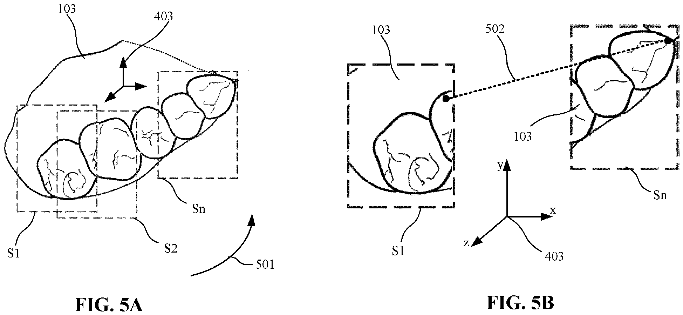

FIGS. 5A and 5B illustrate a distance criterion applied to the generated digital 3D model 103 for transitioning into the second zoom state.

Illustrated in FIG. 5A is the digital 3D model 103 generated by registering the plurality of sub-scans S1, S2,...,Sn. Arrow 501 may indicate direction of movement of the intraoral scanner 101 and the resulting direction of build-up of the digital 3D model 103. Consecutive sub-scans such as sub-scans S1 and S2 may have an overlap based on which the registration of these consecutive sub-scans S1 and S2 is performed.

A distance between the sub-scans S1, S2, ..., Sn registered within the predetermined time may be tracked and compared to a threshold distance. The transitioning to the second zoom state of the digital 3D model 103 may be triggered based on this comparison.

Thus, in an embodiment, the criterion may comprise measuring a distance 502 between furthest points of the part of the digital 3D model 103 generated within the predetermined time and comparing the measured distance 502 to a threshold distance.

The furthest points of the part of the digital 3D model 301 may comprise a point of a first registered sub-scan S1 of the plurality of sub-scans and a point of a last registered sub-scan Sn of the plurality of sub-scans, wherein the plurality of sub-scans are registered within the predetermined time. This is illustrated in FIG. 5B showing the first sub-scan S1 and the last sub-scan Sn registered within the predetermined time.

These two points may be the furthest points between their respective sub-scans S1 and Sn, in the three-dimensional coordinate system 403 of the digital 3D model 103. Instead of the furthest points, any two points may alternatively be used, such as a center of mass or a center of volume points of the corresponding sub-scans S1 and Sn.

The threshold distance may be in a range of 2-10 millimeters, preferably in a range of 4-8 millimeters. If the measured distance 502 is below the selected threshold distance, it may indicate that the motion of the intraoral scanner 101 is sufficiently slow, within the predetermined time, such that the zoom state transition algorithm can be activated.

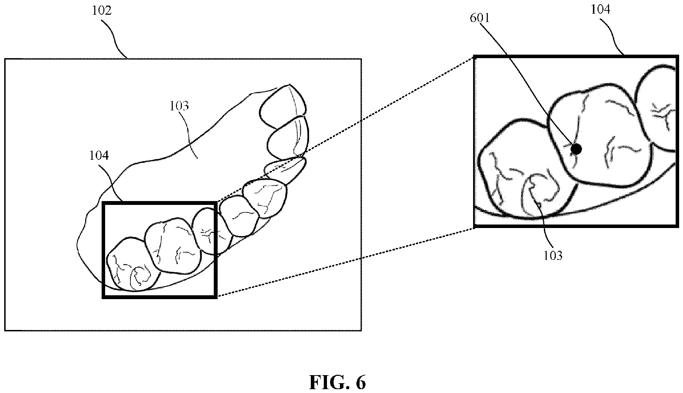

FIG. 6 illustrates a further possible criterion for transitioning into the second zoom state, namely based on monitoring a scan frame property.

The scan frame property may be a speed of movement of the scan frame 104. Thus, in a further embodiment, the criterion may comprise determining that the speed of movement of the scan frame 104 is less than a scan frame speed threshold. This speed reduction may also be observed within the predetermined time.

The scan frame 104 may be displayed on the graphical user interface 102 together with the digital 3D model 103. The position of the scan frame 104 in relation to the digital 3D model 103 displayed on the graphical user interface 102 may correspond to the position of a field of view of the intraoral scanner 101 in relation to the dental object 108 being scanned.

The speed of movement of the scan frame 104 may be a speed of a scan frame center point 601 with respect to the center of mass of the part of the digital 3D model 103 within the scan frame 104. The speed of movement of the scan frame 104 may alternatively be defined between any two points of the scan frame 104 and the part of the digital 3D model 103.

Generally, transitioning to the second zoom state of the digital 3D model 103 may result in simultaneous transitioning from a first zoom state of the scan frame 104 to a second zoom state of the scan frame 104. In that way, the ratio of the scan frame with respect to the part of the digital 3D model 103 within the scan frame 104 may be kept constant.

The second zoom state of the scan frame 104 may correspond to the second zoom state of the digital 3D model 103. This means that the same level of zoom may be applied to both the scan frame 104 and the digital 3D model 103.

Transitioning from the first zoom state of the scan frame 104 to the second zoom state of the scan frame 104 may be realized with a time delay compared with transitioning to the second zoom state of the digital 3D model 103. However, it is preferred that this delay is such that it is not observable by the user.

If determined that the speed of movement of the scan frame 104 is higher than the scan frame speed threshold, transitioning back to the first zoom state of the digital 3D model 103 may be initiated.

In general, the digital 3D model 103 may be configured to rotate around its center of mass or center of volume during the displaying on the graphical user interface 102. The digital 3D model 103 may also be configured to rotate around the center of the scan frame 104 during the displaying on the graphical user interface 102.

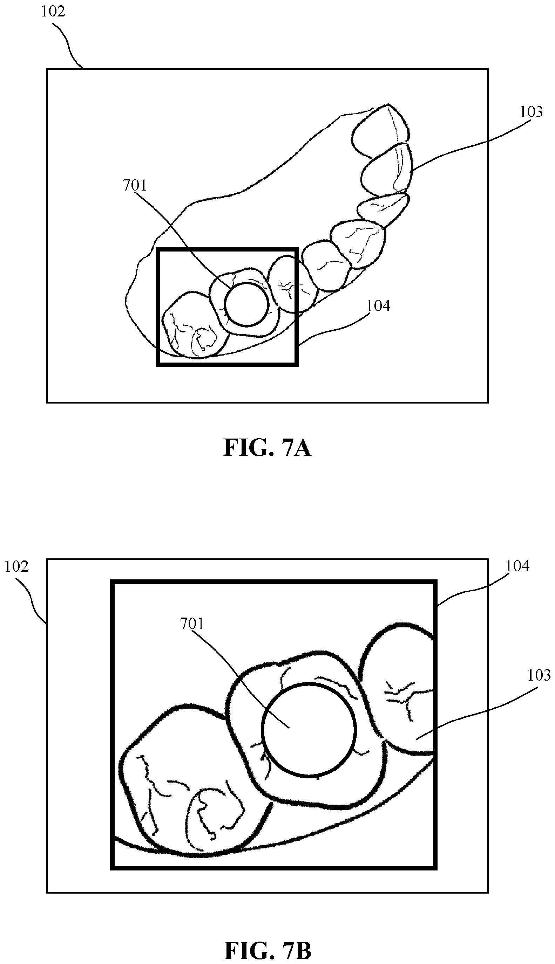

FIGS. 7A and 7B illustrate transitioning into the second zoom state of the digital 3D model 101 based on detecting a tooth preparation for a crown 701.

FIG. 7A shows the graphical user interface 102 displaying the generated digital 3D model 103 and the scan frame 104 representing the current position of the intraoral scanner 101, more specifically its field of view. Within the scan frame 104 the tooth preparation 701 may be detected. The digital 3D model 103 may be in the first zoom state before the tooth preparation 701 is detected.

For the accurate design of a tooth crown it may be necessary to have an accurate scan of the tooth preparation 107. Therefore, it is advantageous to transition to the second zoom state of the digital 3D model 101 when the tooth preparation 701 is detected in the scan frame 104.

The tooth preparation 701 may be automatically detected on the part of the digital 3D model 103 within the scan frame 104 for example through a segmentation algorithm capable of segmenting and classifying individual teeth.

In some cases the user may specify that the tooth preparation 701 will be scanned and separate detection of the tooth preparation 701 is not needed in order to trigger the transition to the second zoom state of the digital 3D model 103.

Detection of the tooth preparation 701 may be used a sole criterion for transitioning to the second zoom state of the digital 3D model 103 or in combination with any other criterion described in the disclosure.

FIG. 7B illustrates the generated digital 3D model 103 in the second zoom state where the tooth preparation 701 may be enlarged compared to the first zoom state of the digital 3D model 103. This advantageously allows the user to capture all details of the tooth preparation 701 for the accurate design of the crown.

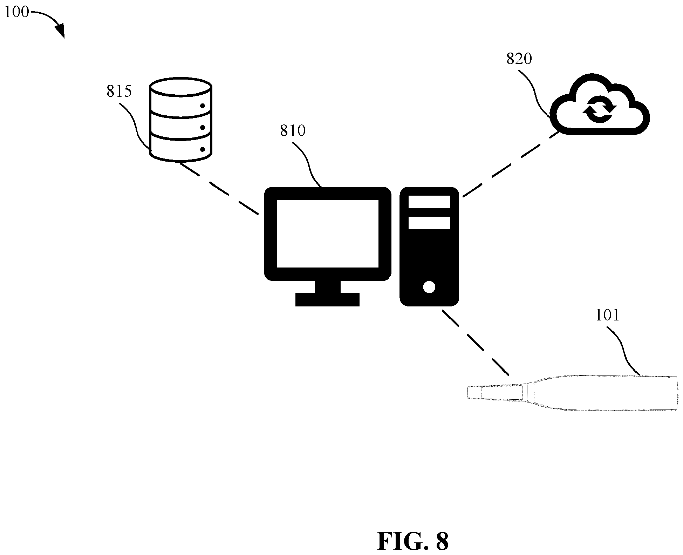

FIG. 8 illustrates the intraoral scanner system 100 which may comprise a computer 810 capable of carrying out any method of the disclosure. The computer 810 may comprise a wired or a wireless interface to a server 815, a cloud server 820 and the wired or wireless intraoral scanner 101. The intraoral scanner 101 may be capable of obtaining light information reflected from the dental object 108.

The intraoral scanner system 100 may comprise a data processing device configured to carry out the method according to one or more embodiments of the disclosure. The data processing device may be a part of the computer 810, the server 815, the cloud server 820, or the intraoral scanner 101.

A non-transitory computer-readable storage medium may be comprised in the dental scanning system 100. The non-transitory computer-readable medium can carry instructions which, when executed by a computer, cause the computer to carry out the method according to one or more embodiments of the disclosure.

The computer-readable storage medium may comprise an optical storage media such as an optical disc, a machine-readable bar code, a USB flash memory, a magnetic storage device, a solid-state electronic storage devices such as random access memory (RAM), or read only memory (ROM), or any other physical device or medium employed to store a computer program. Thereby the embodiments of the disclosure can be utilized on a data processing hardware apparatus, such as a computer system or personal computer, or on an embedded system that employs a dedicated data processing unit, such as a digital signal processing chip.

A computer program product may be embodied in the non-transitory computer-readable storage medium. The computer program product may comprise instructions which, when executed by a computer, cause the computer to perform the method according to any of the embodiments presented herein.

References throughout this disclosure to certain features, advantages, or similar does not imply that all of the features and advantages of the present disclosure should be or are in any single embodiment. Instead, it is to be understood that a specific feature, or characteristic described in connection with an embodiment is included in at least one embodiment of the disclosure. Thus, discussion of the features and advantages, and similar language, throughout this disclosure may, but do not necessarily, refer to the same embodiment.

List of items:

    1. A computer-implemented method comprising:

- obtaining light information reflected from a dental object inside an oral cavity by scanning the dental object with an intraoral scanner;
- generating a digital 3D model of the dental object based on the obtained light information;
- displaying, on a graphical user interface, the digital 3D model in a first zoom state;
- detecting that, as a result of a motion of the intraoral scanner, the generating of the digital 3D model satisfies a criterion;
- transitioning from the first zoom state of the digital 3D model to a second zoom state of the digital 3D model based on the detecting step.

    2. The method according to the previous item 1, wherein the criterion comprises measuring a volume of a part of the digital 3D model generated within a predetermined time and comparing the measured volume to a threshold volume.

    3. The method according to the previous item 2, wherein the volume of the part of the digital 3D model is computed as a volume of a bounding box enclosing the part of the digital 3D model generated within the predetermined time.

    4. The method according to any previous item, wherein a plurality of sub-scans are registered such that a time and a position of each sub-scan of the plurality of sub-scans is recorded.

    5. The method according to the previous item 4, wherein the position of each sub-scan is a center of mass or a center of volume of the each sub-scan.

    6. The method according to any previous item 2-5, wherein comparing the measured volume to the threshold volume comprises determining that the measured volume is less than the threshold volume.

    7. The method according to any previous item 5 or 6, wherein the criterion additionally comprises measuring a speed of the center of mass or a center of volume of each sub-scan of the plurality of sub-scans, and comparing the speed to a threshold speed.

    8. The method according to any previous item 5-7, wherein the criterion additionally comprises measuring an acceleration of the center of mass or a center of volume of each sub-scan of the plurality of sub-scans, and comparing the acceleration to a threshold acceleration.

    9. The method according to the previous item 1, wherein the criterion comprises measuring a distance between furthest points of a part of the digital 3D model generated within the predetermined time and comparing the measured distance to a threshold distance.

    10. The method according to the previous item 9, wherein the furthest points of the part of the digital 3D model correspond to a point of a first registered sub-scan of the plurality of sub-scans and a point of a last registered sub-scan of the plurality of sub-scans, wherein the plurality of sub-scans are registered within the predetermined time.

    11. The method according to the previous item 9 or 10, wherein the threshold distance is in a range of 2-10 millimeters, preferably in a range of 4-8 millimeters.

    12. The method according to the previous item 1, wherein the criterion comprises determining that a speed of movement of a scan frame is less than a scan frame speed threshold.

13. The method according to the previous item 12, wherein the scan frame is displayed on the graphical user interface together with the digital 3D model, wherein the position of the scan frame in relation to the digital 3D model corresponds to the position of a field of view of the intraoral scanner in relation to the dental object being scanned.

14. The method according to the previous item 12 or 13, wherein the speed of movement of the scan frame is a speed of a scan frame center point with respect to a center of mass of the digital 3D model within the scan frame.

15. The method according to any previous item 12-14, further comprising transitioning from a first zoom state of the scan frame to a second zoom state of the scan frame simultaneously with transitioning to the second zoom state of the digital 3D model.

16. The method according to any previous item 12-15, further comprising transitioning from the first zoom state of the scan frame to the second zoom state of the scan frame with a time delay compared with transitioning to the second zoom state of the digital 3D model.

17. The method according to any previous item 12-16, wherein the digital 3D model is configured to rotate around its center of mass or center of volume during the displaying on the graphical user interface.

18. The method according to any previous item 12-16, wherein the digital 3D model is configured to rotate around the center of the scan frame during the displaying on the graphical user interface.

19. The method according to any previous item 15-18, wherein the second zoom state of the scan frame corresponds to the second zoom state of the digital 3D model.

20. The method according to any previous item, wherein the second zoom state is greater than the first zoom state.

21. The method according to any previous item, wherein transitioning from the first zoom state of the digital 3D model to the second zoom state of the digital 3D model enhances visibility of parts of the digital 3D model with missing information.

22. The method according to the previous item 21, wherein the parts of the digital 3D model with missing information are in form of holes in the digital 3D model.

23. The method according to the previous item 22, wherein the holes correspond to a part of the dental object that has not been scanned.

24. The method according to any previous item 22 or 23, wherein the holes in the digital 3D model indicate a part of the dental object requiring additional scanning or re-scanning.

25. The method according to the previous item 21, wherein the parts of the digital 3D model with missing information are colored in a different color compared to the rest of the digital 3D model.

26. The method according to any previous item, wherein transitioning from the first zoom state of the digital 3D model to the second zoom state of the digital 3D model zooms in the digital 3D model on a tooth preparation for a crown.

27. The method according to any previous item, wherein the second zoom state of the digital 3D model is pre-determined.

28. The method according to any previous item, wherein a maximum value of the second zoom state of the digital 3D model is selected such that the scan frame does not become larger than the graphical user interface.

29. The method according to any previous item, further comprising speeding up a zoom animation of the digital 3D model if the digital 3D model is in the second zoom state and if the intraoral scanner is displaced such that its speed of movement exceeds a speed threshold.

30. The method according to any previous item, wherein the second zoom state is determined dynamically.

31. The method according to any previous item 20-30, wherein the second zoom state is a function of a size of a hole in the digital 3D model.

32. The method according to any previous item 20-30, wherein the second zoom state is a function of a size of a tooth within the scan frame of the digital 3D model.

33. The method according to any previous item, further comprising determining that a speed of the tip of the intraoral scanner is higher than the speed threshold and transitioning back to the first zoom state of the digital 3D model.

34. The method according to any previous item, further comprising determining that a speed of movement of the scan frame is higher than the scan frame speed threshold and transitioning back to the first zoom state of the digital 3D model.

35. A computer-implemented method for providing dynamic zoom assistance during scanning process of a dental object is provided, the method comprising:

- obtaining light information reflected from the dental object inside an oral cavity by scanning the dental object with an intraoral scanner;
- generating a digital 3D model of the dental object based on the obtained light information;
- displaying, on a graphical user interface, the digital 3D model in a first zoom state;
- transitioning from the first zoom state of the digital 3D model to the second zoom state of the digital 3D model based on the motion of the intraoral scanner.

36. The method according to the previous item 35, wherein the change in motion of the intraoral scanner is a speed of a tip of the intraoral scanner.

37. The method according to the previous items 35 or 36, wherein the motion of the intraoral scanner is measured by a sensor comprised in the intraoral scanner.

38. The method according to the previous item 37, wherein the sensor is an encoder.

38. A computer-implemented method for providing dynamic zoom assistance during scanning process of a dental object is provided, the method comprising:

- obtaining light information reflected from the dental object inside an oral cavity by scanning the dental object with an intraoral scanner;
- generating a digital 3D model of the dental object based on the obtained light information;
- displaying, on the graphical user interface, the digital 3D model in a first zoom state;
- transitioning from the first zoom state of the digital 3D model to a second zoom state of the digital 3D model based on detecting a voice control signal.

39. The method according to the previous item 38, wherein the voice control signal is registered by a microphone comprised in the intraoral scanner or in an intraoral scanner system.

40. A computer-implemented method for providing dynamic zoom assistance during scanning process of a dental object is provided, the method comprising:

- obtaining light information reflected from the dental object inside an oral cavity by scanning the dental object with an intraoral scanner;
- generating a digital 3D model of the dental object based on the obtained light information;
- displaying, on a graphical user interface, the digital 3D model in a first zoom state;
- detecting a tooth preparation within the generated digital 3D model;
- transitioning from the first zoom state of the digital 3D model to a second zoom state of the digital 3D model based on the detecting step.

41. The method according to the previous item 40, wherein detecting of the tooth preparation comprises applying a segmentation algorithm on a registered sub-scan.

42. A dental scanning system comprising:

- an intraoral scanner for scanning a dental object inside an oral cavity, the intraoral scanner comprising:
- a projector unit for projecting a pattern of light onto a surface of the dental object;
- an image sensor for acquiring a plurality of images in response to illuminating the surface of the dental object;
- a processor operatively coupled to the intraoral scanner, the processor configured to perform the steps according to any item 1-41.

42. A dental scanning system comprising:

- an intraoral scanner for scanning a dental object inside an oral cavity, the intraoral scanner comprising:
- a projector unit for projecting a pattern of light onto a surface of the dental object;
- an image sensor for acquiring a plurality of images in response to illuminating the surface of the dental object;
- a processor operatively coupled to the intraoral scanner, the processor configured for:

    - obtaining light information reflected from the dental object inside the oral cavity;
    - generating a digital 3D model of the dental object based on the obtained light information;
    - displaying, on the graphical user interface, the digital 3D model in a first zoom state;
    - detecting that, as a result of a motion of the intraoral scanner, the generating of the digital 3D model satisfies a criterion;
- transitioning from the first zoom state of the digital 3D model to a second zoom state of the digital 3D model based on the detecting step.

43. A computer program product comprising instructions which, when executed by a computer, cause the computer to perform the method according to any previous item 1-41.

44. A non-transitory computer-readable medium comprising instructions which, when executed by a computer, cause the computer to carry out the method according to any previous item 1-41.

45. A computer-readable storage medium carrying instructions which, when executed by a computer, cause the computer to carry out the method according to any previous item 1-41.

46. A computer-implemented method for providing dynamic zoom assistance during scanning process of a dental object, the method comprising:

- obtaining light information reflected from the dental object inside an oral cavity by scanning the dental object with an intraoral scanner,
- generating a digital 3D model of the dental object based on the obtained light information,
- displaying, on a graphical user interface, the digital 3D model in a first zoom state,
- transitioning from the first zoom state of the digital 3D model to a second zoom state of the digital 3D model based on detecting a user engaging a button on the intraoral scanner.

**Claims**

1. A computer-implemented method for providing dynamic zoom assistance during scanning process of a dental object, the method comprising:

   - obtaining light information reflected from a dental object inside an oral cavity by scanning the dental object with an intraoral scanner;
   - generating a digital 3D model of the dental object based on the obtained light information;
   - displaying, on a graphical user interface, the digital 3D model in a first zoom state;
   - detecting that, as a result of motion of the intraoral scanner, the generating of the digital 3D model satisfies a criterion;
   - transitioning from the first zoom state of the digital 3D model to a second zoom state of the digital 3D model based on the detecting step.

2. The method according to the previous claim 1, wherein the criterion comprises measuring a volume of a part of the digital 3D model generated within a predetermined time and comparing the measured volume to a threshold volume.

3. The method according to the previous claim 2, wherein the volume of the part of the digital 3D model is computed as a volume of a bounding box enclosing the part of the digital 3D model generated within the predetermined time.

4. The method according to the previous claim 2, wherein the volume of the part of the digital 3D model is determined by calculating signed volumes of tetrahedrons which are formed by joining the vertices of facets of the part of the digital 3D model with an arbitrary point and summing up the calculated volumes.

5. The method according to the previous claim 1, wherein the criterion comprises measuring a distance between furthest points of a part of the digital 3D model generated within a predetermined time and comparing the measured distance to a threshold distance.

6. The method according to the previous claim 5, wherein the furthest points of the part of the digital 3D model correspond to a point of a first registered sub-scan of a plurality of sub-scans and a point of a last registered sub-scan of the plurality of sub-scans, wherein the plurality of sub-scans are registered within the predetermined time.

7. The method according to the previous claim 5 or 6, wherein the threshold distance is in a range of 2-10 millimeters, preferably in a range of 4-8 millimeters.

8. The method according to the previous claim 1, wherein the criterion comprises determining that a speed of movement of a scan frame is less than a scan frame speed threshold, wherein the scan frame is a 2D frame displayed on the graphical user interface representing a current position of the intraoral scanner.

9. The method according to the previous claim 8, further comprising determining that the speed of movement of the scan frame is higher than the scan frame speed threshold and transitioning back to the first zoom state of the digital 3D model.

10. The method according to any previous claim, wherein transitioning from the first zoom state of the digital 3D model to the second zoom state of the digital 3D model zooms in on a part of the digital 3D model with missing information.

**11.** The method according to the previous claim 10, wherein the part of the digital 3D model with missing information is in form of a hole in the digital 3D model.

**12.** The method according to any previous claim, wherein the criterion further comprises detecting a tooth preparation for a crown, a filling and/or a dental condition lesion.

**13.** A dental scanning system comprising:

- an intraoral scanner for scanning a dental object inside an oral cavity, the intraoral scanner comprising:
- a projector unit for projecting a pattern of light onto a surface of the dental object;
- an image sensor for acquiring a plurality of images in response to illuminating the surface of the dental object;
- a processor operatively coupled to the intraoral scanner, wherein the processor is configured for performing the method according to any previous claim 1-12.

**14.** A computer-readable medium comprising instructions which, when executed by a computer, cause the computer to carry out the method according to any previous item 1-12.

**15.** A computer program product comprising instructions which, when executed by a computer, cause the computer to perform the method according to any previous item 1-12.

**FIG. 1**

200

201

Obtaining light information reflected from a
dental object by an intraoral scanner

202

Generating a digital 3D model of the dental
object based on the obtained light information

203

Displaying, on a GUI, the digital 3D model in
a first zoom state

204

Detecting that, as a result of motion of the
intraoral scanner, generating of the digital 3D
model satisfies a criterion

205

Transitioning from the first zoom state of the
digital 3D model to a second zoom state of
the digital 3D model

**FIG. 2**

**FIG. 3A**

**FIG. 3B**

**FIG. 4A**

**FIG. 4B**

**FIG. 5A**

**FIG. 5B**

**FIG. 6**

**FIG. 7A**

**FIG. 7B**

100

815

810

820

101

**FIG. 8**

Europäisches
Patentamt
European
Patent Office
Office européen
des brevets

# EUROPEAN SEARCH REPORT

**Application Number**

EP 24 19 3264

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/192488 A1 (SABINA MICHAEL [US] ET AL) 18 June 2020 (2020-06-18)<br>* paragraph [0021] *<br>* paragraph [0028] *<br>* paragraph [0044] - paragraph [0047] *<br>* paragraph [0035] *<br>* paragraph [0052]; figure 4 *<br>* paragraph [0053] *<br>* paragraph [0070] *<br>----- | 1-15 | INV.<br>G06T7/521<br>G06T7/55<br>A61C9/00<br>G06F3/01<br>G06T17/00 |
| X | US 2024/023800 A1 (FRIDMAN EDI [IL]) 25 January 2024 (2024-01-25)<br>* paragraph [0100] - paragraph [0105] *<br>----- | 1-15 | |
| X | US 2021/196152 A1 (SAPHIER OFER [IL] ET AL) 1 July 2021 (2021-07-01)<br>* paragraph [0006] - paragraph [0007] *<br>* paragraph [0036] *<br>----- | 1-15 | |
| X | WO 2021/211871 A1 (ALIGN TECHNOLOGY INC [US]) 21 October 2021 (2021-10-21)<br>* paragraph [0317] *<br>----- | 1-15 | TECHNICAL FIELDS SEARCHED (IPC) |
| X | US 2018/168781 A1 (KOPELMAN AVI [US] ET AL) 21 June 2018 (2018-06-21)<br>* paragraph [0073] - paragraph [0074] *<br>----- | 1-15 | G06T<br>A63F<br>A61C<br>G06F |

The present search report has been drawn up for all claims

| Place of search | Date of completion of the search | Examiner |
|---|---|---|
| Munich | 19 December 2024 | Garton, Paul |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 19 3264

This annex lists the patent family members relating to the patent documents cited in the above-mentioned European search report.
The members are as contained in the European Patent Office EDP file on
The European Patent Office is in no way liable for these particulars which are merely given for the purpose of information.

19-12-2024

| Patent document cited in search report | | Publication date | Patent family member(s) | | Publication date |
|---|---|---|---|---|---|
| US 2020192488 A1 | | 18-06-2020 | CN | 107427345 A | 01-12-2017 |
| | | | CN | 113397739 A | 17-09-2021 |
| | | | EP | 3265021 A1 | 10-01-2018 |
| | | | EP | 3865087 A1 | 18-08-2021 |
| | | | US | 2016259515 A1 | 08-09-2016 |
| | | | US | 2019011996 A1 | 10-01-2019 |
| | | | US | 2020192488 A1 | 18-06-2020 |
| | | | US | 2022291754 A1 | 15-09-2022 |
| | | | US | 2024248545 A1 | 25-07-2024 |
| | | | WO | 2016142817 A1 | 15-09-2016 |
| US 2024023800 A1 | | 25-01-2024 | NONE | | |
| US 2021196152 A1 | | 01-07-2021 | NONE | | |
| WO 2021211871 A1 | | 21-10-2021 | CN | 115884727 A | 31-03-2023 |
| | | | EP | 4136569 A1 | 22-02-2023 |
| | | | US | 2021321872 A1 | 21-10-2021 |
| | | | US | 2021353152 A1 | 18-11-2021 |
| | | | US | 2021353153 A1 | 18-11-2021 |
| | | | US | 2021353154 A1 | 18-11-2021 |
| | | | WO | 2021211871 A1 | 21-10-2021 |
| US 2018168781 A1 | | 21-06-2018 | US | 2018168780 A1 | 21-06-2018 |
| | | | US | 2018168781 A1 | 21-06-2018 |
| | | | US | 2020237486 A1 | 30-07-2020 |
| | | | US | 2021121271 A1 | 29-04-2021 |
| | | | US | 2021298878 A1 | 30-09-2021 |
| | | | US | 2023141733 A1 | 11-05-2023 |
| | | | WO | 2018112273 A2 | 21-06-2018 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2442720 B1